# EUROPEAN PATENT APPLICATION

(11) **EP 4 631 568 A1**
(43) Date of publication of application: **15.10.2025**
(21) Application number: 25169100.2
(22) Date of filing: 08.04.2025
(51) Int. Cl.: A61N 5/10

(54) **METHOD AND APPARATUS TO FACILITATE OPTIMIZING A RADIATION TREATMENT PLAN**

(30) Priority: 10.04.2024 US 202418631352
(71) Applicant: Siemens Healthineers International AG, 6312 Steinhausen (CH)
(72) Inventor: HAKALA, Mikko, 05200 Rajamaki (FI); CZEIZLER, Elena, 00390 Helsinki (FI); SUHONEN, Pauli, 00760 Helsinki (FI)
(74) Representative: Mathisen & Macara LLP

(57) **Abstract**

A control circuit identifies at least one field geometry parameter value 201 and then determines 203 dosimetric robustness for the at least one field geometry parameter value to produce a robustness assessment. The control circuit can then determine 205 whether the robustness assessment is satisfactory, and when true, optimize 206 a radiation treatment plan using the at least one field geometry parameter value.

## Description

### COPYRIGHT NOTICE

A portion of the disclosure of this patent document contains material which is subject to copyright protection. The copyright owner has no objection to the facsimile reproduction by anyone of the patent document or the patent disclosure, as it appears in the Patent and Trademark Office patent file or records, but otherwise reserves all copyright rights whatsoever.

### TECHNICAL FIELD

These teachings relate generally to treating a patient's planning target volume with energy pursuant to an energy-based treatment plan and more particularly to optimizing an energy-based treatment plan.

### BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

In external beam radio therapy (photons or protons), beam placement is one parameter that usually needs to be set and which may influence the plan quality. Fields are typically placed as a function of the planner's experience, templated solutions, or in some cases using beam geometry optimizers.

### SUMMARY

In one aspect the present invention provides a method to facilitate optimizing a radiation treatment plan as defined in claim 1. Optional features are specified in the dependent claims.

In another aspect the present invention provides an apparatus to facilitate optimizing a radiation treatment plan as defined in claim 11. Optional features are specified in the dependent claims.

### BRIEF DESCRIPTION OF DRAWINGS

The above needs are at least partially addressed through provision of the method and apparatus to facilitate optimizing a radiation treatment plan described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings;
FIG. 3 comprises a schematic view as configured in accordance with various embodiments of these teachings;
FIG. 4 comprises a graph as configured in accordance with various embodiments of these teachings; and
FIG. 5 comprises a graph as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Generally speaking, pursuant to these various embodiments, a control circuit identifies at least one field geometry parameter value and then determines dosimetric robustness for the at least one field geometry parameter value to produce a robustness assessment. The control circuit can then determine whether the robustness assessment is satisfactory, and when true, optimize a radiation treatment plan using the at least one field geometry parameter value.

By one approach, identifying the at least one field geometry parameter value can comprise at least one of accessing a default field geometry parameter value and/or receiving user input specifying the field geometry parameter value. Examples of field geometry parameter values include, but are not limited to, a starting point gantry angle of a volumetric modulated arc therapy field, a stopping point gantry angle of a volumetric modulated arc therapy field, an avoidance sector start angle in a volumetric modulated arc therapy radiation treatment plan, an avoidance sector stop angle in a volumetric modulated arc therapy radiation treatment plan, a gantry angle of an intensity modulated radiation therapy field, a collimator angle, a patient support surface angle, and/or a patient restraint angle.

By one approach, determining the dosimetric robustness for the at least one field geometry parameter value to produce the robustness assessment can comprise optimizing radiation treatment plans at a plurality of field geometry parameter values that are closely proximal to, but not equal to, the at least one field geometry parameter value.

By one approach, the plurality of field geometry parameter values that are closely proximal to, but not equal to, the at least one field geometry parameter value can comprise at least two field geometry parameter values that are on opposing sides of the at least one field geometry parameter value. By one approach, the foregoing can comprise a first plurality of field geometry parameter values that are on a first side of the at least one field geometry parameter value and a second plurality of field geometry parameter values that are on a second side of the at least one field geometry parameter value, which second side is on an opposite side of the at least one field geometry parameter value from the first side.

These teachings are highly flexible in practice and will accommodate a variety of modifications and/or supplemental features. For example, when the robustness assessment is partially, but not wholly satisfactory, these teachings will nevertheless accommodate optimizing the radiation treatment plan using a field geometry parameter value that is modified from the at least one field geometry parameter value. In such a case, and by one approach, the field geometry parameter value that is modified from the at least one field geometry parameter value can be constrained to fall within a range of field geometry parameter values that were previously tested when determining the dosimetric robustness.

As another example of the flexibility of these teachings, these teachings will accommodate identifying at least one second field geometry parameter value for a second field geometry parameter than is different than the field geometry parameter for the at least one field geometry parameter. In this case, determining the dosimetric robustness can comprise determining the dosimetric robustness for both the at least one field geometry parameter value and the at least one second field geometry parameter value.

Sensitivity of a treatment planning system with respect to one or more field geometry parameters refers to the situation when small variations of these parameter values within a (typically small) range of field geometry variations lead to significant changes in some metrics related to the generated plan and/or dose distribution. The metrics used to analyze the sensitivity of a system to such variations of field geometry parameter values can include any of a variety of dosimetric metrics such as ones based on the clinical goals or the optimization cost function(s) as well as other plan related metrics such as monitor units or treatment delivery time. For example, a given treatment platform/system can be viewed as being sensitive to a certain field geometry parameter upon observing significant changes of one or more optimization cost function values when making small modifications in the values of that parameter. When such small variations in the values of field geometry parameters do not lead to significant variations in the considered metrics, we may say that the system is robust against such changes.

Small variations in the foregoing regards can occur due to uncertainties that can arise with respect to, for example, patient positioning and/or patient anatomy at the time of a given treatment session. Absent these teachings, such uncertainty can lead to critical under or overdosage when the field geometry happens to include a non-robust region where a slight change in patient geometry can place the radiation fields off their optimal position and lead to non-optimal dosimetric alterations of the radiation treatment plan.

The present teachings can help to identify robust field geometries that are less susceptible to result in such dosing inaccuracies due to uncertainties in patient anatomy or positioning.

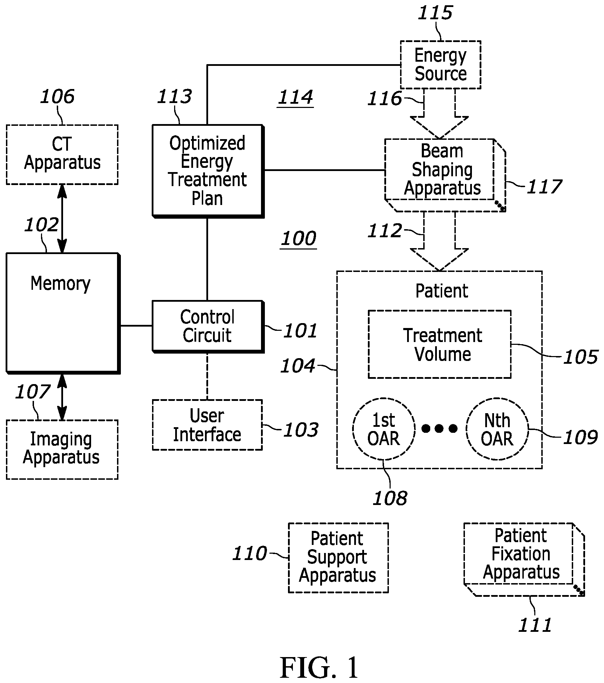

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient and information regarding a particular radiation treatment platform as described herein, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

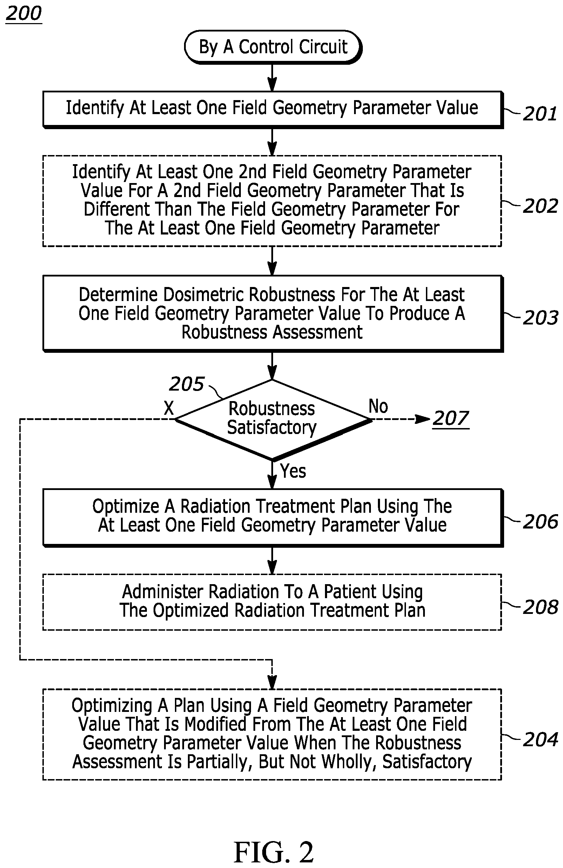

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate treating a particular patient with therapeutic radiation using a particular radiation treatment platform per that optimized radiation treatment plan.

At block 201, this process 200 provides for the control circuit 101 identifying at least one field geometry parameter value as corresponds to a radiation treatment plan to be optimized. This identification can be achieved in any of a variety of ways. For example, by one approach, the control circuit 101 accesses a default field geometry parameter value that is stored in the aforementioned memory 102. This default value might constitute, for example, a value that is an average value for this particular parameter in the experience of one or more treatment facilities. By another approach, the control circuit 101 may receive user input (via, for example, the aforementioned user interface 103) that specifies the field geometry parameter value. These teachings will accommodate other approaches in these regards as well as desired. As one example in these regards, a properly configured and trained machine learning model could provide the field geometry parameter value.

These teachings will also accommodate any of a variety of field geometry parameters. By one general approach, the field geometry parameter value can comprise a value for a gantry position. As noted above, a gantry is a mechanism that can provide selective movement of a radiation source while administering radiation to a patient. More particularly, a gantry is a movable frame that houses the linac head, which generates high-energy radiation beams to be directed at a target volume such as a tumor. A typical gantry is designed to rotate around the patient, who is positioned on a treatment table, allowing for precise targeting of the radiation from multiple angles while sparing surrounding healthy tissue.

A non-limiting list of potentially useful field geometry parameter values can include a starting point gantry angle of a volumetric modulated arc therapy field, a stopping point gantry angle of a volumetric modulated arc therapy field, an avoidance sector start angle in a volumetric modulated arc therapy radiation treatment plan, an avoidance sector stop angle in a volumetric modulated arc therapy radiation treatment plan, a gantry angle of an intensity modulated radiation therapy field, a collimator angle, a patient support surface angle, and/or a patient restraint angle, to note but a few.

If desired, and as illustrated at optional block 202, this process 200 can also provide for identifying at least one second field geometry parameter value for a second field geometry parameter that is different than the field geometry parameter for the aforementioned at least one field geometry parameter.

At block 203, the control circuit 101 determines dosimetric robustness for the at least one field geometry parameter value to produce a robustness assessment. When there are two or more additional field geometry parameter values (for additional field geometry parameters), this determination of the dosimetric robustness can comprise determining dosimetric robustness for both the at least one field geometry parameter value and the at least one second field geometry parameter value to produce a corresponding multi-dimensional robustness assessment.

By one approach, determining the dosimetric robustness for the at least one field geometry parameter value can comprise optimizing a plurality of radiation treatment plans at a plurality of different field geometry parameter values that are closely proximal to, but not equal to, the at least one field geometry parameter value. In many application settings it may be useful if there are at least two field geometry parameter values that are each on opposing sides of the at least one field geometry parameter value. In other words, that there is at least one field geometry parameter value corresponding to one side of the identified field geometry parameter value and at least one other field geometry parameter value that corresponds to the other side of the identified field geometry parameter value. If desired, there can be a plurality of values on one side of the identified field geometry parameter value and another plurality of other values on the other side of the identified field geometry parameter value.

To determine dosimetric robustness, these teachings will accommodate calculating a radiation dosing that corresponds to each of the aforementioned values, both at the identified value and at other nearby locations as well. The more the dosing results of the nearby values match the dosing result for the identified value, the more robust the result.

A specific level of robustness can be specified by the user if desired. Robustness might be defined, for example, by requiring that none of the dosing values for the nearby field geometry parameter values vary from the dosing value for the identified field geometry parameter value by more than some specified amount, such as, for example, anywhere within the range of 0 percent to 5 percent, 10 percent, or some other upper range limit of choice.

The control circuit 101 can determine, at block 205, whether the determined dosimetric robustness corresponding to the one or more identified field geometry parameter value is sufficiently robust. The criterion by which such sufficiency is judged can be selected, for example, by the user. It may be noted that certain clinical sites (for example, breast cancer cases or pancreas cancer cases) may be more sensitive to small variations in the beam geometry parameters than other clinical sites. In such cases, the criterion by which one determines a sufficient level of robustness can be higher and more selective than the criterion by which one determines robustness sufficiency in less-sensitive application settings.

Should the robustness be judged insufficient, the process 200 will accommodate whatever corresponding action or actions 207 might be desired. The latter action may include, for example, simply notifying the user of this situation, automatically re-identifying different field geometry parameter values and re-doing this process 200 until a satisfactory result is obtained, or any other action of choice.

When the determined dosimetric robustness is satisfactory, at block 206 the control circuit 101 can optimize a radiation treatment plan using the identified (one or more) field geometry parameter value(s), reasonably secure that the resultant plan will not only achieve good results if administered exactly per the identified values but also in the event there are variations in those values at the time of administering the radiation treatment plan.

At optional block 208, this process 200 will accommodate administering radiation to a patient using the aforementioned optimized radiation treatment plan. By one approach, feedback information from the administered plan may be used to inform changes to, for example, a definition or criterion by which dosimetric robustness is determined and/or used to determine a predicted satisfactory result.

It is possible that the determination of whether the dosimetric robustness is satisfactory will yield a less determinative result. Instead of being clearly satisfactory or clearly unsatisfactory, the result may indicate that robustness is partially, though not wholly, satisfactory. In this case, and as illustrated at optional block 204, this process 200 will accommodate optimizing the radiation treatment plan using a field geometry parameter value that is modified from the identified field geometry parameter value(s). For example, it may be acceptable to select a field geometry parameter value that was within the range of field geometry parameter values that were tested when determining dosimetric robustness at block 203.

Further details that comport with these teachings will now be presented. It will be understood that the specific details of these examples are intended to serve an illustrative purpose and are not intended to suggest any particular limitations with respect to these teachings.

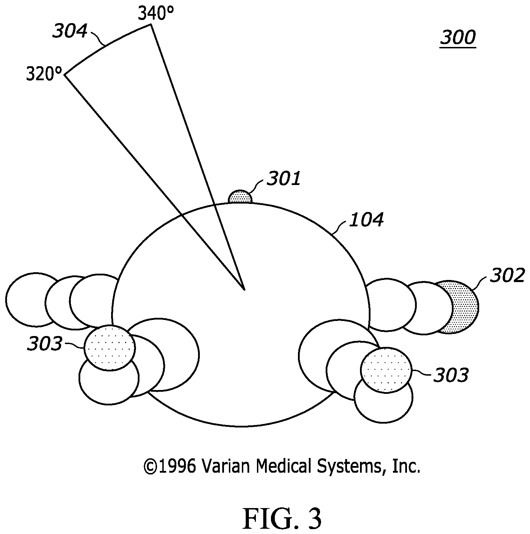

FIG. 3 presents a schematic view of a patient 104. (The patient 104 is represented in this view by a copyrighted schematic characterization of a human patient (generally colored green on a user's display), with the small circle 301 (typically colored red on a user's display) corresponding to the patient's nose, the circle 302 (typically also colored red on a user's display) at the end of the patient's left arm corresponding to the patient's left hand, and the two circles denoted by reference numeral 303 (typically colored blue on a user's display) corresponding to the patient's toes. This schematic representation provides a convenient and useful way to depict a patient's torso and appendages without reference to gender for the purposes of planning a radiation treatment.) This image also presents a range for a single field geometry parameter 304 to be scanned for field geometry robustness (in this illustrative example, the parameter comprising a volumetric modulated arc therapy start gantry angle). In this illustrative example the identified field geometry parameter value is 330 degrees and the range of angle values to be investigated is within ten degrees on either side of that identified value.

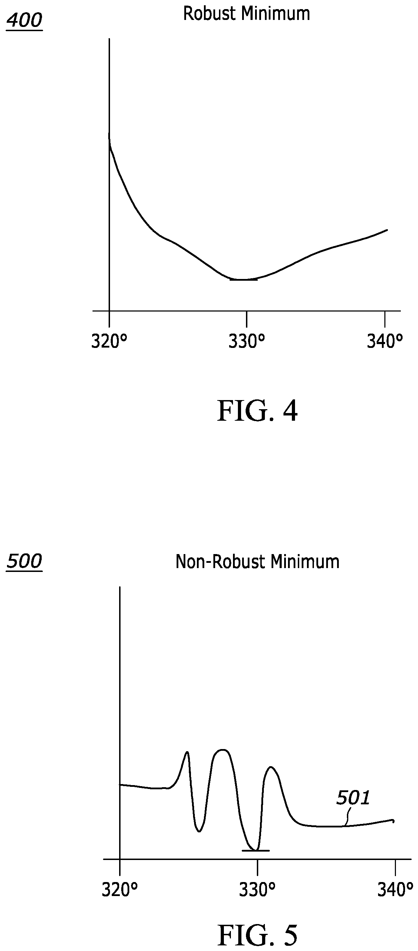

FIG. 4 presents a graph 400 depicting a situation in which the relevant cost function varies smoothly when optimization is carried out as a function of the gantry angle within the scanned range represented in FIG. 3 (i.e., from 320 degrees on the gantry to 340 degrees). Although the dosimetric results may vary somewhat, the degree of those variations does not vary greatly over small angular distances on either side of 330 degrees and therefore one might conclude that the value of 330 degrees is a robust value.

FIG. 5 presents a graph 500 depicting a situation in which the cost function is highly varying around 330 degrees until about 332 degrees and which also has a plateau 501 from 332 degrees to 340 degrees. By one approach, these teachings may suggest simply rejecting 330 degrees as an insufficiently robust identified field geometry parameter value. By another approach, these teachings may suggest using the value of 336 degrees, as that angle is in the middle of the aforementioned plateau 501 and therefore represents a robust alternative.

By one approach, these teachings will accommodate also monitoring clinical goal achievements and non-achievements within the tested parametric region of field geometry. Such an approach can provide an additional quantitative measure to assess the robustness of an initial field geometry selection.

Generally speaking, the foregoing process 200 uses a cost function of optimization as a proxy for monitoring whether the chosen field geometry is in a smooth or a highly varying region in terms of dosimetric uncertainty. That said, a useful metric in these regards can be other than the optimization cost. Examples include but are not limited to the value of the target's minimum dose, the number of monitor units for a particular field, and so forth. Generally speaking, the applicable metric can be anything that reflects the efficacy of the plan.

Generally speaking, these teachings can accommodate using and leveraging essentially any radiation treatment plan quality index that is not dependent explicitly on field geometry setup as an indicator of field geometry robustness. Accordingly, any quantitative value that can be derived from the result of (i.e., after) optimization can also be used as a proxy to monitor the robustness of the optimized plan in terms of the field geometry parameters.

It may also be noted that these teachings will accommodate using more than one metric (for example, two metrics, three metrics, or more as desired) to monitor the robustness of a particular selected field geometry. For example, one metric can be the value of the optimizer's cost function and another metric the value derived from clinical goals. As a simple example, the plan may be non-robust with respect to the optimizer cost function, but robust with respect to some quality function derived from clinical goals. If a treatment facility decides that the plan must be robust with respect to all robustness indicators, this kind of a plan would not qualify as acceptable.

It will be appreciated that these teachings can be employed as an independent method that can help select and define field geometry during a planning session. In lieu of the foregoing, or in combination therewith, these teachings can also be employed as an additional stage in a corresponding beam geometry optimization algorithm of choice.

Further aspects of the disclosure are provided by the subject matter of the following clauses:
Clause 1. A method to facilitate optimizing a radiation treatment plan, the method comprising: by a control circuit: identifying at least one field geometry parameter value; determining dosimetric robustness for the at least one field geometry parameter value to produce a robustness assessment; determining whether the robustness assessment is satisfactory; optimizing the radiation treatment plan using the at least one field geometry parameter value when the robustness assessment is satisfactory.
Clause 2. The method of any preceding clause or combination of clauses wherein identifying the at least one field geometry parameter value comprises at least one of: accessing a default field geometry parameter value; receiving user input specifying the field geometry parameter value.
Clause 3. The method of any preceding clause or combination of clauses wherein determining dosimetric robustness for the at least one field geometry parameter value to produce the robustness assessment comprises optimizing radiation treatment plans at a plurality of field geometry parameter values that are closely proximal to, but not equal to, the at least one field geometry parameter value.
Clause 4. The method of any preceding clause or combination of clauses wherein the plurality of field geometry parameter values that are closely proximal to, but not equal to, the at least one field geometry parameter value comprise at least two field geometry parameter values that are on opposing sides of the at least one field geometry parameter value.
Clause 5. The method of any preceding clause or combination of clauses wherein the at least two field geometry parameter values that are on opposing sides of the at least one field geometry parameter value comprise a first plurality of field geometry parameter values that are on a first side of the at least one field geometry parameter value and a second plurality of field geometry parameter values that are on a second side of the at least one field geometry parameter value, which second side is on an opposite side of the at least one field geometry parameter value from the first side.
Clause 6. The method of any preceding clause or combination of clauses wherein the at least one field geometry parameter value comprises a value for a gantry position.
Clause 7. The method of any preceding clause or combination of clauses wherein the at least one field geometry parameter value comprises at least one of: a starting point gantry angle of a volumetric modulated arc therapy field; a stopping point gantry angle of a volumetric modulated arc therapy field; an avoidance sector start angle in a volumetric modulated arc therapy radiation treatment plan; an avoidance sector stop angle in a volumetric modulated arc therapy radiation treatment plan; a gantry angle of an intensity modulated radiation therapy field; a collimator angle; a patient support surface angle; a patient restraint angle.
Clause 8. The method of any preceding clause or combination of clauses further comprising: optimizing the radiation treatment plan using a field geometry parameter value that is modified from the at least one field geometry parameter value when the robustness assessment is partially, but not wholly, satisfactory.
Clause 9. The method of any preceding clause or combination of clauses wherein the field geometry parameter value that is modified from the at least one field geometry parameter value falls within a range of field geometry parameter values that were tested when determining the dosimetric robustness.
Clause 10. The method of any preceding clause or combination of clauses further comprising: identifying at least one second field geometry parameter value for a second field geometry parameter than is different than the field geometry parameter for the at least one field geometry parameter; and wherein determining the dosimetric robustness for the at least one field geometry parameter value to produce a robustness assessment comprises determining the dosimetric robustness for both the at least one field geometry parameter value and the at least one second field geometry parameter value to produce a robustness assessment.
Clause 11. An apparatus to facilitate optimizing a radiation treatment plan, the apparatus comprising: a control circuit configured to: identify at least one field geometry parameter value; determine dosimetric robustness for the at least one field geometry parameter value to produce a robustness assessment; determine whether the robustness assessment is satisfactory; optimize the radiation treatment plan using the at least one field geometry parameter value when the robustness assessment is satisfactory.
Clause 12. The apparatus of any preceding clause or combination of clauses wherein identifying the at least one field geometry parameter value comprises at least one of: accessing a default field geometry parameter value; receiving user input specifying the field geometry parameter value.
Clause 13. The apparatus of any preceding clause or combination of clauses wherein determining dosimetric robustness for the at least one field geometry parameter value to produce the robustness assessment comprises optimizing radiation treatment plans at a plurality of field geometry parameter values that are closely proximal to, but not equal to, the at least one field geometry parameter value.
Clause 14. The apparatus of any preceding clause or combination of clauses wherein the plurality of field geometry parameter values that are closely proximal to, but not equal to, the at least one field geometry parameter value comprise at least two field geometry parameter values that are on opposing sides of the at least one field geometry parameter value.
Clause 15. The apparatus of any preceding clause or combination of clauses wherein the at least two field geometry parameter values that are on opposing sides of the at least one field geometry parameter value comprise a first plurality of field geometry parameter values that are on a first side of the at least one field geometry parameter value and a second plurality of field geometry parameter values that are on a second side of the at least one field geometry parameter value, which second side is on an opposite side of the at least one field geometry parameter value from the first side.
Clause 16. The apparatus of any preceding clause or combination of clauses wherein the at least one field geometry parameter value comprises a value for a gantry position.
Clause 17. The apparatus of any preceding clause or combination of clauses wherein the at least one field geometry parameter value comprises at least one of: a starting point gantry angle of a volumetric modulated arc therapy field; a stopping point gantry angle of a volumetric modulated arc therapy field; an avoidance sector start angle in a volumetric modulated arc therapy radiation treatment plan; an avoidance sector stop angle in a volumetric modulated arc therapy radiation treatment plan; a gantry angle of an intensity modulated radiation therapy field; a collimator angle; a patient support surface angle; a patient restraint angle.
Clause 18. The apparatus of any preceding clause or combination of clauses further comprising: optimizing the radiation treatment plan using a field geometry parameter value that is modified from the at least one field geometry parameter value when the robustness assessment is partially, but not wholly, satisfactory.
Clause 19. The apparatus of any preceding clause or combination of clauses wherein the field geometry parameter value that is modified from the at least one field geometry parameter value falls within a range of field geometry parameter values that were tested when determining the dosimetric robustness.
Clause 20. The apparatus of any preceding clause or combination of clauses further comprising: identifying at least one second field geometry parameter value for a second field geometry parameter than is different than the field geometry parameter for the at least one field geometry parameter; and wherein determining the dosimetric robustness for the at least one field geometry parameter value to produce a robustness assessment comprises determining the dosimetric robustness for both the at least one field geometry parameter value and the at least one second field geometry parameter value to produce a robustness assessment.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

## Claims

1. A method to facilitate optimizing a radiation treatment plan, the method comprising:
by a control circuit:
identifying at least one field geometry parameter value;
determining dosimetric robustness for the at least one field geometry parameter value to produce a robustness assessment;
determining whether the robustness assessment is satisfactory;
optimizing the radiation treatment plan using the at least one field geometry parameter value when the robustness assessment is satisfactory.

2. The method of claim 1 wherein identifying the at least one field geometry parameter value comprises at least one of:
accessing a default field geometry parameter value;
receiving user input specifying the field geometry parameter value.

3. The method of claim 1 or 2 wherein determining dosimetric robustness for the at least one field geometry parameter value to produce the robustness assessment comprises optimizing radiation treatment plans at a plurality of field geometry parameter values that are closely proximal to, but not equal to, the at least one field geometry parameter value.

4. The method of claim 3 wherein the plurality of field geometry parameter values that are closely proximal to, but not equal to, the at least one field geometry parameter value comprise at least two field geometry parameter values that are on opposing sides of the at least one field geometry parameter value.

5. The method of claim 4 wherein the at least two field geometry parameter values that are on opposing sides of the at least one field geometry parameter value comprise a first plurality of field geometry parameter values that are on a first side of the at least one field geometry parameter value and a second plurality of field geometry parameter values that are on a second side of the at least one field geometry parameter value, which second side is on an opposite side of the at least one field geometry parameter value from the first side.

6. The method of any one of claims 1 to 5 wherein the at least one field geometry parameter value comprises a value for a gantry position.

7. The method of any one of claims 1 to 5 wherein the at least one field geometry parameter value comprises at least one of:
a starting point gantry angle of a volumetric modulated arc therapy field;
a stopping point gantry angle of a volumetric modulated arc therapy field;
an avoidance sector start angle in a volumetric modulated arc therapy radiation treatment plan;
an avoidance sector stop angle in a volumetric modulated arc therapy radiation treatment plan;
a gantry angle of an intensity modulated radiation therapy field;
a collimator angle;
a patient support surface angle;
a patient restraint angle.

8. The method of any one of claims 1 to 7 further comprising:
optimizing the radiation treatment plan using a field geometry parameter value that is modified from the at least one field geometry parameter value when the robustness assessment is partially, but not wholly, satisfactory.

9. The method of claim 8 wherein the field geometry parameter value that is modified from the at least one field geometry parameter value falls within a range of field geometry parameter values that were tested when determining the dosimetric robustness.

10. The method of any one of claims 1 to 9 further comprising:
identifying at least one second field geometry parameter value for a second field geometry parameter than is different than the field geometry parameter for the at least one field geometry parameter;
and wherein determining the dosimetric robustness for the at least one field geometry parameter value to produce a robustness assessment comprises determining the dosimetric robustness for both the at least one field geometry parameter value and the at least one second field geometry parameter value to produce a robustness assessment.

11. An apparatus to facilitate optimizing a radiation treatment plan, the apparatus comprising:
a control circuit configured to:
identify at least one field geometry parameter value;
determine dosimetric robustness for the at least one field geometry parameter value to produce a robustness assessment;
determine whether the robustness assessment is satisfactory;
optimize the radiation treatment plan using the at least one field geometry parameter value when the robustness assessment is satisfactory.

12. The apparatus of claim 11 wherein identifying the at least one field geometry parameter value comprises at least one of:
accessing a default field geometry parameter value;
receiving user input specifying the field geometry parameter value.

13. The apparatus of claim 11 or 12 wherein determining dosimetric robustness for the at least one field geometry parameter value to produce the robustness assessment comprises optimizing radiation treatment plans at a plurality of field geometry parameter values that are closely proximal to, but not equal to, the at least one field geometry parameter value.

14. The apparatus of claim 13 wherein the plurality of field geometry parameter values that are closely proximal to, but not equal to, the at least one field geometry parameter value comprise at least two field geometry parameter values that are on opposing sides of the at least one field geometry parameter value, wherein, optionally, the at least two field geometry parameter values that are on opposing sides of the at least one field geometry parameter value comprise a first plurality of field geometry parameter values that are on a first side of the at least one field geometry parameter value and a second plurality of field geometry parameter values that are on a second side of the at least one field geometry parameter value, which second side is on an opposite side of the at least one field geometry parameter value from the first side.

15. The apparatus of any one of claims 11 to 14 wherein the control circuit is configured to perform the method of any one of claims 6 to 10.
